(19) Europäisches Patentamt
European Patent Office
Office européen des brevets

(11) EP 1 076 718 B1

(12) # FASCICULE DE BREVET EUROPEEN

(45) Date de publication et mention de la délivrance du brevet:
**02.05.2002 Bulletin 2002/18**

(21) Numéro de dépôt: **99918034.2**

(22) Date de dépôt: **10.05.1999**

(51) Int Cl.7: **C12P 41/00**, C12P 13/04, C07D 233/80

(86) Numéro de dépôt international:
**PCT/FR99/01098**

(87) Numéro de publication internationale:
**WO 99/58706 (18.11.1999 Gazette 1999/46)**

(54) **NOUVEAU PROCEDE DE PREPARATION D'INTERMEDIAIRES DE SYNTHESE**
VERFAHREN ZUR HERSTELLUNG VON ZWISCHENPRODUKTEN FÜR SYNTHESE
NOVEL METHOD FOR PREPARING SYNTHESIS INTERMEDIATES

(84) Etats contractants désignés:
**AT BE CH DE DK ES FI FR GB GR IE IT LI LU NL PT SE**

(30) Priorité: **14.05.1998 FR 9806339**

(43) Date de publication de la demande:
**21.02.2001 Bulletin 2001/08**

(73) Titulaire: **Aventis CropScience S.A.**
**69009 Lyon (FR)**

(72) Inventeurs:
- **BONTOUX, Marie-Claude**
**F-38200 Luzinay (FR)**
- **FAVRE-BULLE, Olivier**
**F-69003 Lyon (FR)**

(56) Documents cités:
**EP-A- 0 178 553** **EP-A- 0 629 616**
**US-A- 5 552 318**

- **KAPTEIN, BERNARD ET AL: "Enzymic resolution of.alpha.,.alpha.-disubstituted.alpha.-ami no acid esters and amides" TETRAHEDRON: ASYMMETRY (1993), 4(6), 1113-16 CODEN: TASYE3;ISSN: 0957-4166, 1993, XP002091821 cité dans la demande**
- **KEIL, OLIVER ET AL: "New hydantoinases from thermophilic microorganisms. Synthesis of enantiomerically pure D-amino acids" TETRAHEDRON: ASYMMETRY (1995), 6(6), 1257-60 CODEN: TASYE3;ISSN: 0957-4166, 1995, XP002091822**
- **CHEMICAL ABSTRACTS, vol. 117, no. 21, 23 novembre 1992 (1992-11-23) Columbus, Ohio, US; abstract no. 212925, PIRKLE, WILLIAM H. ET AL: "Preparation of.alpha.-substituted.alpha.-amino acids of high enantiomeric purity" XP002091823 & CHIRALITY (1992), 4(5), 302-7 CODEN: CHRLEP;ISSN: 0899-0042,1992,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 076 718 B1

## Description

### Domaine de l'invention

La présente invention concerne un nouveau procédé de préparation d'intermédiaires de synthèse optiquement actifs pour la fabrication de composés chimiques. Plus particulièrement, la présente invention concerne un procédé d'enrichissement énantiomérique de dérivés d'α-phénylalanine.

### État de la technique

Dans de nombreux domaines comme par exemple la pharmacie et l'agrochimie, les composés chimiques actifs sont de plus en plus complexes et comprennent très souvent un ou plusieurs centres d'asymétrie. La plupart de ces produits commercialisés le sont sous forme de racémique, c'est-à-dire que toutes les formes énantiomériques et/ou diastéréo-isomériques sont présentes dans la matière active. Or, dans de très nombreux cas, seule l'une des formes optiquement active possède l'activité recherchée, alors que les autres isomères optiques sont inactifs voire peuvent aller à l'encontre de l'effet désiré et même présenter des risques pour les mammifères et/ou l'environnement. Il est donc important de pouvoir disposer de procédés de synthèse chimiques ou biochimiques (enzymatiques par exemple) conduisant au seul isomère désiré. De tels procédés sont par exemple les synthèses asymétriques ou, lorsque le produit de départ est un mélange d'énantiomères, les procédés dits d'enrichissement énantiomérique.

Des procédés d'enrichissement énantiomérique par catalyse enzymatique sont connus dans la littérature. Parmi ceux-ci, on peut citer par exemple, la publication de B. Kaptein et coll. (*Tetrahedron Asymetry, 4*(6), (1993), 1113-1116), dans laquelle est décrite l'hydrolyse d'un ester éthylique par la lipase de foie de porc ; cependant la sélectivité est très mauvaise, c'est-à-dire que l'excès énantiomérique du produit n'est pas satisfaisant.

Le brevet WO-A-96/12035 divulgue l'accès par catalyse enzymatique à des esters d'acide carboxylique possédant un excès énantiomérique élevé. Le catalyseur utilisé est choisi parmi bactéries, lipases et protéases. Ici encore, les excès énantiomériques observés sont relativement faibles, et notamment dans le cas des énantiomères *S*.

Le brevet US 5,552,318 décrit un procédé d'enrichissement de l'ester éthylique de la DL-homophénylalanine à l'aide d'enzymes, mais les excès énantiomériques observés sont très variables. Enfin, la demande de brevet EP 178 553) décrit un enrichissement énantiomérique d'acides aminés par action d'α-chymotripsine.

Un objet de la présente invention consiste à fournir un procédé d'enrichissement énantiomérique par catalyse enzymatique conduisant à un composé ayant un excès énantiomérique élevé.

Un objet de la présente invention consiste à fournir un procédé de préparation par catalyse enzymatique d'un composé substantiellement énantiomériquement pur.

Un autre objet de la présente invention consiste à fournir un procédé de préparation, par catalyse enzymatique, à partir d'un mélange d'énantiomères, de l'énantiomère *S* substantiellement pur, ou de l'énantiomère *R* substantiellement pur.

Un autre objet de la présente invention consiste à fournir un procédé d'enrichissement énantiomérique par catalyse enzymatique à partir d'un mélange d'énantiomères d'esters.

Un autre objet de l'invention consiste à fournir un procédé d'enrichissement énantiomérique par catalyse enzymatique d'un mélange d'enantiomères d'esters en composés substantiellement énantiomériquement purs, avec un rendement élevé.

Il a maintenant été découvert que tous ces buts peuvent être atteints en totalité ou en partie grâce au procédé selon l'invention, dont la description est présentée ci-dessous.

### Brève description de l'invention

La présente invention fournit un nouveau procédé d'enrichissement énantiomérique d'esters de l'α-phénylalanine, caractérisé en ce que l'on met en contact un mélange d'énantiomères d'esters de l'α-phénylalanine avec un catalyseur enzymatique comprenant des enzymes non purifiés, ou partiellement ou totalement purifiés. Ces enzymes peuvent être utilisés libres ou absorbés ou immobilisés sur des supports organiques ou inorganiques selon des méthodes bien connues de l'homme de l'art.

Les composés substantiellement énantiomériquement purs ainsi obtenus peuvent servir comme intermédiaires de synthèse dans l'élaboration de matières actives chirales utiles en thérapeutique ou en agriculture. A titre d'exemple les énantiomères d'esters de l'α-phénylalanine sont utilisés comme intermédiaires dans la préparation de certaines 2-imidazoline-5-ones et 2-imidazoline-5-thiones fongicides décrites dans le brevet EP-A-0 629 616.

**Description détaillée de l'invention**

La présente invention concerne donc un nouveau procédé de préparation d'esters de l'α-phénylalanine substantiellement énantiomériquement purs de formules (I) et (II) :

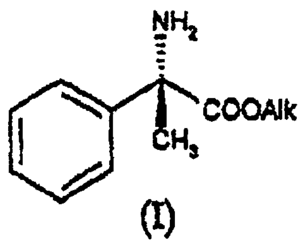

(I)

Isomère *S*

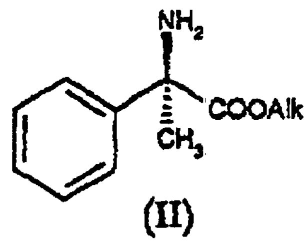

(II)

Isomère *R*

dans lesquelles Alk représente un radical alkyle linéaire ou ramifié comportant de 1 à 6 atomes de carbone, tel que par exemple le radical méthyle, éthyle, n-propyle, n-butyle, n-pentyle, n-hexyle, iso-propyle, iso-butyle, iso-pentyle, iso-hexyle, tertio-butyle, tertio-pentyle, tertio-hexyle, néo-pentyle ou néo-hexyle,

procédé caractérisé en ce que l'on met en présence un mélange d'énantiomères d'asters de l'α-phénylalanine avec une lipase, une estérase ou une protéase, sous forme libre, absorbée ou immobilisée, à l'exception de l'enzeyme PLE (Pig Liver Esterase).

Par mélange d'énantiomères, on entend un mélange en proportions égales ou différentes de l'isomère *S* de formule (I) et de l'isomère *R* de formule (II) de l'ester de l'α-phénylalanine de formule (A) :

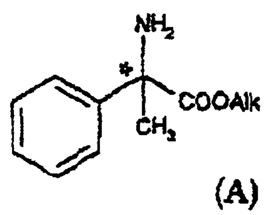

(A)

dans laquelle Alk représente un radical alkyle linéaire ou ramifié comportant de 1 à 6 atomes de carbone, tel que celui défini dans les formules (I) et (II), et l'astérisque indique le centre d'asymétrie de l'ester.

Le procédé selon l'invention permet d'obtenir des énantiornères substantiellement purs, soit de configuration *S* (ester de formule (I)), soit de configuration *R* (ester de formule (II)).

Le terme "substantiellement pur" signifie que l'excès énantiomérique de l'énantiomère considéré est supérieur à 80%, plus particulièrement supérieur à 90%. Certains enzymes utilisés dans le procédé selon l'invention conduisent également à un excès énantiomérique de 100% ; c'est-à-dire que, dans ce cas, l'énantiomère obtenu est pur, l'autre forme énantiomérique n'étant pas décelable.

Par excès énantiomérique, on entend le ratio de l'excès de l'énantiomère désiré par rapport à l'énantiomère non désiré.

Ce ratio est calculé selon l'une des équations suivantes :

$$\%\ \text{e.e.(S)} = \frac{[S] - [R]}{[R] + [S]} \times 100 \qquad \%\ \text{e.e.(R)} = \frac{[R] - [S]}{[R] + [S]} \times 100$$

dans lesquelles :

% e.e.(S) représente l'excès énantiomérique en isomère *S*,
% e.e.(R) représente l'excès énantiomérique en isomère *R*,
[*S*] représente la concentration en isomère *S*, et
[*R*] représente la concentration en isomère *R*.

Le procédé de l'invention met en oeuvre des enzymes, en particulier des lipases, des estérases et des protéases.

Les protéases permettent d'accéder à l'énantiomère S de formule (I) définie ci-dessus ; les lipases ou les estérases permettent d'accéder à l'énantiomère R de formule (II) définie ci-dessus.

Les enzymes appropriés pour le procédé de l'invention sont plus particulièrement choisis parmi les enzymes suivants disponibles sur le marché :

| Enzyme | Fournisseur |
|---|---|
| Candidia Antartica (immob.) | Novo |
| L1754 | Sigma |
| Lipase A Rhizopus | Amano |
| Lipase CE | Amano |
| Lipase Chirazyme E1 | Boehringer |
| Lipase Chirazyme L1 | Boehringer |
| Lipase Chirazyme L2 | Boehringer |
| Lipase Chirazyme L3 | Boehringer |
| Lipase Chirazyme L4 | Boehringer |
| Lipase Chirazyme L5 | Boehringer |
| Lipase Chirazyme L6 | Boehringer |
| Lipase Chirazyme L7 | Boehringer |
| Lipase Chirazyme L8 | Boehringer |
| Lipase GC | Amano |
| Lipase L3001 | Sigma |
| Lipase L3126 | Sigma |
| Lipase pancréatique L115P | Sigma |
| Liver Acetone Powder L9627 | Sigma |
| Liver porcine L8521 | Sigma |
| MAP 10 | Amano |
| Protéase Biofeed | Novo |
| Protéase Prozyme | Amano |
| Protéase Subtilisine | Boehringer |
| Protéase Subtilisine A | Novo |
| Protéase Trypsine | Sigma |
| PS | Amano |
| Protéase P5147 | Sigma |
| Protéase P4032 (aspergillus) | Sigma |

Parmi les protéases, on préférera les protéases d'aspergillus (comme la Prozyme de Amano ou la protéase P4032 de Sigma), la Subtilisine de Boehringer ou de Novo, le Biofeed de Novo et la trypsine.

Parmi les lipases et les estérases, on préférera plus particulièrement les lipases Chirazyme L2, L5, L7 et E1 de Boehringer.

Pour le procédé selon l'invention, on utilisera plus préférentiellement encore une protéase d'aspergillus ou une lipase ou une estérase de porc qui sont spécifiques de l'une ou l'autre des deux formes énantiomères.

Les protéases d'aspergillus conduisent spécifiquement à l'isomère *S* de formule (I) ci-dessus définie et parmi celles-ci une protéase tout particulièrement préférée dans le procédé de l'invention est la Prozyme de la société Amano.

Les lipases et estérases de porc conduisent quant à elles spécifiquement à l'énantiomère *R* de formule (II) ci-dessus définie.

Le procédé d'enrichissement énantiomérique selon l'invention consiste donc à mettre en contact un mélange d'esters de formule (A) définie ci-dessus avec une quantité catalytique d'enzyme, choisi parmi les protéases si l'on souhaite obtenir l'énantiomère *S* de formule (I) définie ci-dessus, ou choisi parmi les lipases ou estérases si l'on souhaite obtenir l'énantiomère *R* de formule (II) définie ci-dessus.

Cette réaction peut être conduite dans des conditions diverses et variées qui sont bien connues de l'homme du métier spécialiste des réactions biocatalytiques, et connues notamment dans la littérature, y compris dans les "Chemical Abstracts" et les bases de données informatiques.

De manière générale, le procédé est réalisé dans un milieu ayant un pH compris entre 5 et 10. De préférence, la réaction a lieu au pH optimal défini par le fournisseur de l'enzyme.

La réaction est conduite à une température généralement comprise entre 4°C et 70°C. De préférence, la température est la température optimale définie par le fournisseur de l'enzyme.

La durée de la réaction à une incidence directe sur le taux de transformation et est généralement comprise entre 1 et 120 heures, plus préférentiellement entre 10 et 60 heures, selon la température de réaction et le nature de l'enzyme utilisé.

Les valeurs de pH, de température, et de durée de réaction données ici sont celles généralement retenues, mais il est bien entendu que les conditions opératoires seront adaptées à chaque réaction spécifique.

Le procédé selon l'invention peut être mis en oeuvre en présence ou en absence d'eau, en présence ou en absence d'un ou plusieurs solvants ou en présence d'eau et d'un ou plusieurs solvants. D'une manière générale, ces solvants sont compatibles avec l'activité enzymatique et sont choisis parmi ceux qui solubilisent l'ester de formule (A). Selon un aspect tout particulièrement préféré de l'invention, ces solvants sont choisis parmi les solvants aromatiques. Plus préférentiellement encore, les solvants sont par exemple le toluène ou le monochlorobenzène.

Le ratio volumique de la quantité de solvant utilisé par rapport à la quantité d'eau est généralement compris entre 5% et 90%, de préférence entre 20% et 80%.

Le procédé décrit ci-dessus est réalisé, de façon générale, avec l'enzyme et le mélange d'énantiomères se trouvant en phase homogène ou hétérogène.

Une variante du procédé selon l'invention consiste à mettre en oeuvre en quantité correspondante et appropriée l'enzyme immobilisé sur un support, suivant des méthodes bien connues de l'homme du métier, telles que par exemple celles décrites dans K. H. Lee *et coll.*, *J. Chem. Tech. Biotechnol.*, *54*, (1992), 375-382.

Les esters de formule (I) et (II) définis précédemment obtenus selon le procédé de l'invention trouvent une application tout particulièrement intéressante en tant qu'intermédiaires de synthèse dans l'élaboration de matières actives chirales utiles notamment en thérapeutique ou en agriculture.

Par exemple, les esters de formule (I) et (II) peuvent être utilisés comme intermédiaires dans la préparation de certaines 2-imidazoline-5-ones et 2-imidazoline-5-thiones fongicides décrites dans le brevet EP-A-0 629 616 de formule (F) :

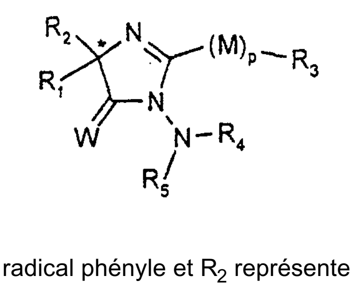

(F)

dans laquelle $R_1$ représente le radical phényle et $R_2$ représente le radical méthyle et

- W représente un atome d'oxygène ou de soufre, ou un groupe S=O ;
- M représente un atome d'oxygène ou de soufre, ou un radical $CH_2$, éventuellement halogéné ;

- p est un nombre entier égal à 0 ou 1 ;
- $R_3$ représente :

  - un hydrogène ou un radical $C_1$-$C_2$ alkyle éventuellement halogéné, lorsque p égale 0 ou $(M)_p$ est un radical $CH_2$,
  - un radical $C_1$-$C_2$ alkyle éventuellement halogéné, lorsque $(M)_p$ représente un atome d'oxygène ou de soufre ;

- $R_4$ représente :

  - l'atome d'hydrogène, ou
  - un radical alkyle contenant de 1 à 6 atomes de carbone, ou
  - un radical alkoxyalkyle, alkylthioalkyle, haloalkyle, cyanoalkyle, thiocyanatoalkyle, alcényle ou alcynyle contenant de 2 à 6 atomes de carbone, ou
  - un radical dialkylaminoalkyle, alkoxycarbonylalkyle, ou N-alkylcarbamoylalkyle contenant de 3 à 6 atomes de carbone, ou
  - un radical N,N-dialkylcarbamoylalkyle contenant de 4 à 8 atomes de carbone, ou
  - un radical aryle, comprenant phényle, naphtyle, thiényle, furyle, pyridyle, pyrimidyle, pyridazinyle, pyrazinyle, benzothiényle, benzofuryle, quinolinyle, isoquinolinyle, ou méthylène dioxyphényle, éventuellement substitué par 1 à 3 groupements choisis parmi $R_6$, ou
  - un radical arylalkyle, aryloxyalkyle, arylthioalkyle ou arylsulfonylalkyle, les termes aryle et alkyle ayant les définitions données ci-dessus ;

- $R_5$ représente :

  - l'hydrogène, ou un radical alkyle, haloalkyle, alkylsulfonyle, haloalkylsulfonyle contenant de 1 à 6 atomes de carbone ou,
  - un radical alkoxyalkyle, alkylthioakyle, acyle, alcényle, alcynyle, haloacyle, alkoxycarbonyle, haloalkoxycarbonyle, alkoxyalkylsulfonyle, cyanoalkylsulfonyle contenant de 2 à 6 atomes de carbone ou
  - un radical alkoxyalkoxycarbonyle, alkylthioalkoxycarbonyle, cyanoalkoxycarbonyle contenant de 3 à 6 atomes de carbone ou,
  - le radical formyle ou un radical cycloalkyle, alkoxyacyle, alkylthioacyle, cyanoacyle, alcénylcarbonyle, alcynylcarbonyle contenant de 3 à 6 atomes de carbone ou,
  - un radical cycloalkylcarbonyle contenant de 4 à 8 atomes de carbone ou,
  - un radical phényle; arylalkylcarbonyle, notamment phénylacétyle et phénylpropionyle, arylcarbonyle, notamment benzoyle, éventuellement substitué par 1 à 3 groupes parmi $R_6$, thiénylcarbonyle, furylcarbonyle, pyridylcarbonyle, benzyloxycarbonyle, furfuryloxycarbonyle, tetrahydrofurfuryloxycarbonyle, thiénylméthoxycarbonyle, pyridylméthoxycarbonyle, phénoxycarbonyle ou phénylthiolcarbonyle, le radical phényle étant lui-même éventuellement substitué par 1 à 3 groupement choisis parmi $R_6$, alkylthiolcarbonyle, haloalkylthiolcarbonyle, alkoxyalkylthiolcarbonyle, cyanoalkylthiolcarbonyle, benzylthiolcarbonyle, furfurylthiolcarbonyle, tétrahydrofurfurylthiolcarbonyle, thiénylméthylthiolcarbonyle, pyridylméthylthiolcarbonyle, ou arylsulfonyle ou
  - un radical carbamoyle éventuellement mono ou disubstitué par :

    - un groupe alkyle ou haloalkyle contenant de 1 à 6 atomes de carbone,
    - un groupe cycloalkyle, alcényle ou alcynyle contenant de 3 à 6 atomes de carbone,
    - un groupe alkoxyalkyle, alkylthioalkyle ou cyanoalkyle contenant de 2 à 6 atomes de carbone ou,
    - un phényle éventuellement substitué par 1 à 3 groupement $R_6$ ;

  - un groupement sulfamoyle éventuellement mono ou disubstitué par :

    - un groupe alkyle ou haloalkyle contenant de 1 à 6 atomes de carbone,
    - un groupe cycloalkyle, alcényle ou alcynyle contenant de 3 à 6 atomes de carbone,
    - un groupe alkoxyalkyle, alkylthioalkyle ou cyanoalkyle de 2 à 6 atomes de carbone ou
    - un phényle éventuellement substitué par 1 à 3 groupement $R_6$ ;

  - un groupe alkylthioalkylsulfonyle contenant de 3 à 8 atomes de carbone ou cycloalkylsulfonyle contenant de 3 à 7 atomes de carbone ;
  - $R_4$ et $R_5$ pris ensemble peuvent également former avec l'atome d'azote auquel ils sont attachés un groupe pyrrolidino, pipéridino, morpholino ou pipérazino éventuellement substitué par un radical alkyle contenant de

1 à 3 atomes de carbone.

- $R^6$ représente :
  - un atome d'halogène ou
  - un radical alkyle, haloalkyle, alkoxy, haloalkoxy, alkylthio, haloalkylthio ou alkylsulfonyle contenant de 1 à 6 atomes de carbone ou
  - un radical cycloalkyle, halocycloalkyle, alcényloxy, alcynyloxy, alcénylthio, alcynylthio contenant de 3 à 6 atomes de carbone ou
  - le groupe nitro ou cyano ou
  - un radical amino éventuellement mono ou disubstitué par un radical alkyle ou acyle contenant de 1 à 6 atomes de carbone ou alkoxycarbonyle contenant de 2 à 6 atomes de carbone,
  - un radical phényle, phénoxy ou pyridyloxy, ces radicaux étant éventuellement substitués par 1 à 3 groupements, identiques ou différents, choisis parmi $R_7$,

- $R_7$ représente :
  - un atome d'halogène choisi parmi le fluor, le chlore, le brome, l'iode ou,
  - un radical alkyle contenant de 1 à 6 atomes de carbone, ou
  - un radical alkoxy ou alkylthio contenant de 1 à 6 atomes de carbone ou,
  - un radical haloalkoxy ou haloalkylthio contenant de 1 à 6 atomes de carbone ou,
  - un radical nitrile ou nitro ;

Le procédé de préparation des composés de formule (F) peut être représenté par exemple par le schéma suivant :

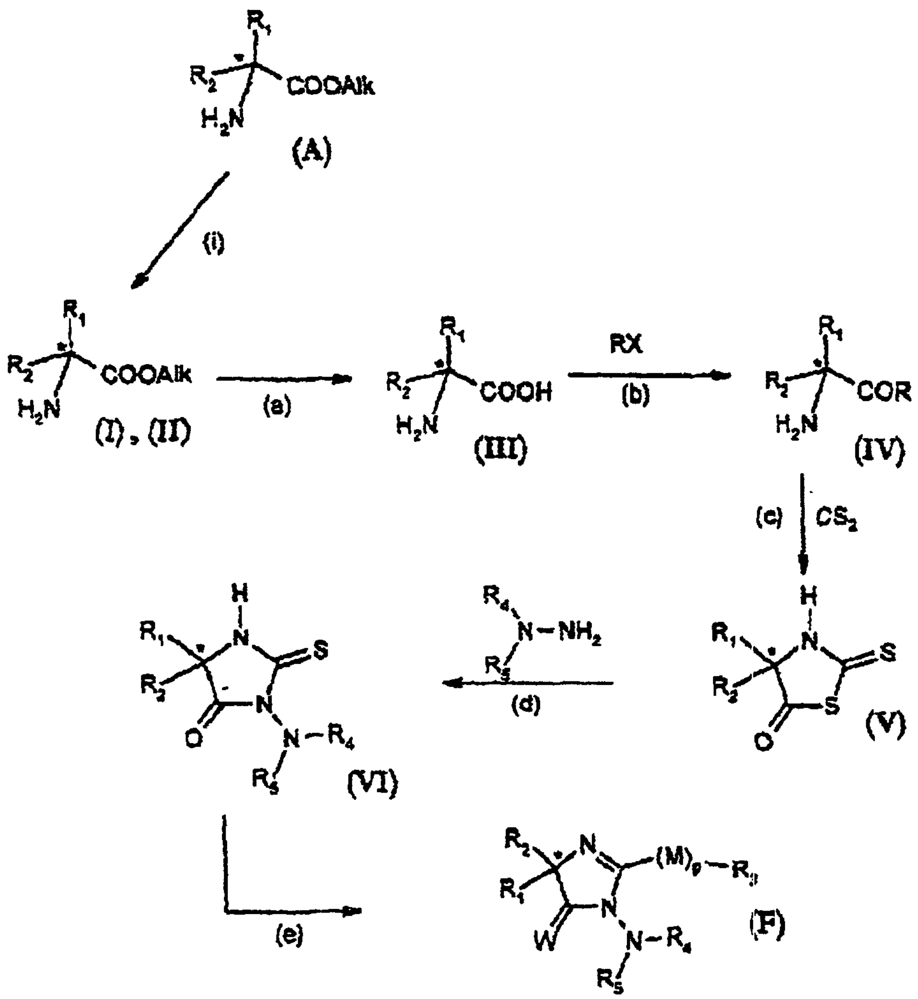


schéma dans lequel, les radicaux Alk, $R_1$, $R_2$, $R_3$, $R_4$, $R_5$, M, p et W sont tels que définis précédemment, R représente un radical hydroxy, alkoxy contenant de 1 à 6 atomes de carbone, benzyloxy, un radical amino, alkylamino ou dialkylamino, un radical alkylamino contenant de 1 à 6 atomes de carbone, et X représente un groupement partant tel qu'un atome d'halogène, choisi parmi chlore, brome et iode, ou un radical sulfate, ou alkylsulfonyloxy ou arylsulfonyloxy.

Dans le schéma précédent

- l'étape (i) est le procédé de la présente invention et est exemplifié dans la suite de la présente description ;
- l'étape (a) est une étape classique de saponification ;
- l'étape (b) est une réaction d'estérification classique ;
- l'étape (c) est une réaction de cyclisation réalisée par addition de sulfure de carbone, en milieu basique et connue de l'homme du métier et décrite par exemple dans la demande de brevet internationale WO98/03490 ;
- l'étape (d) est réalisée par addition d'une amine de formule $N(R_4R_5)$-$NH_2$, en présence d'un catalyseur (amine tertiaire par exemple), comme décrit par exemple dans la demande de brevet internationale WO98/03490 ;
- l'étape (c) est finalement réalisée par addition d'un composé de formule $R_3$-X, pour conduire au composé -S-$R_3$ correspondant, puis éventuellement oxydé, selon des techniques classiques pour conduire aux différents degrés d'oxydation de l'atome de soufre, ou encore traité par un composé de formule $R_3$-OH pour conduire au composé -O-$R_3$ correspondant. Ces différentes réactions sont bien connues de l'homme du métier et sont par exemple décrites dans la demande de brevet EP-A-0 629 616.

Il est bien entendu que lorsque le radical R représente le radical Oalk, les étapes (2) et (b) sont superflues et peuvent ne pas être conduites dans ce schéma de synthèse générale.

Le procédé global de synthèse des composés de formule (F), à partir d'acides aminés de formule (A) via les intermédiaires de formules (I) et (II), est nouveau et à ce titre est compris dans le champ de la présente invention.

La mise en oeuvre du procédé d'enrichissement énantiomérique selon l'invention est illustrée par les exemples représentatifs suivants ; ces exemples n'apportent en aucun cas de limitation aux caractéristiques de la présente invention.

## EXEMPLE 1

Dans un tube pèse-matières, agité magnétiquement, sont chargés successivement 250 mg de protéase d'aspergillus (Amano Prozyme 6) et 5 ml de tampon phosphate 100 mM contenant 50 mM d'un mélange racémique d'esters méthyliques de l'α-phénylalanine.

Après 17 heures de réaction à 30°C, l'excès énantiomérique de l'ester résiduel est mesuré par CLHP (Chromatographie Liquide à Haute Pression) chirale. On trouve un ester résiduel de configuration S avec un excès énantiomérique de 99,4%.

## EXEMPLES 2 à 5

En répétant l'exemple 1 ci-dessus et en remplaçant la protéase Prozyme 6 de Amano par d'autres enzymes, les résultats suivants sont obtenus :

| *Exemple* | *Enzyme* | *Configuration de l'ester résiduel* | *Excès énantiomérique* |
|---|---|---|---|
| 2 | Subtilisine (Boehringer) | S | 79,5 |
| 3 | Subtilisine a (Novo) | S | 80,5 |
| 4 | Protéase P4032 | S | 100 |
| 5 | Estérase E1 (Boehringer) | R | 71 |

## EXEMPLE 6

Dans un tube pèse-matières, agité magnétiquement, sont chargés successivement 0,2 g de protéase Prozyme, 2,5 ml de tampon phosphate 100 mM (pH 7), 2,5 ml de toluène et 0,223 g d'un mélange racémique d'esters méthyliques de l'α-phénylalanine.

Après 120 heures de réaction à 30°C, la phase organique est récupérée et évaporée. L'ester méthylique résiduel est repris dans l'éluant de la CLHP chirale puis injecté sur une colonne chirale AGP (Interchim). L'excès énantiomérique de l'ester résiduel de configuration S est de 80%.

## Revendications

1. Procédé de préparation d'esters de l'α-phénylalanine substantiellement énantiomériquement purs de formules (I) et (II) :

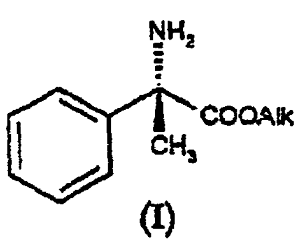

(I)

Isomère *S*

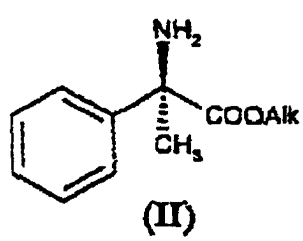

(II)

Isomère *R*

dans lesquelles Alk représente un radical alkyle linéaire ou ramifié comportant de 1 à 6 atomes de carbone, **caractérisé en ce que** l'on met en présence un mélange d'énantiomères d'esters de l'α-phénylalanine avec un enzyme qui est une lipase, une protéase ou une estérase, à l'exception de l'enzeyme PLE (Pig Liver Esterase).

2. Procédé selon la revendication 1 dans lequel "Alk" représente le radical méthyle.

3. Procédé selon la revendication 1 dans lequel "Alk" représente le radical éthyle.

4. Procédé selon l'une des revendications 1 à 3 **caractérisé en ce que** l'ester substantiellement énantiomériquement pur est de configuration *S* et l'enzyme utilisé est une protéase.

5. Procédé selon la revendication 4 dans lequel la protéase est choisie parmi le Biofeed, la Prozyme, la Subtilisine, la Subtilisine A, la Trypsine, la protéase PS, P5147 et P4032.

6. Procédé selon l'une des revendications 4 ou 5 dans lequel la protéase est une protéase d'aspergillus, Prozyme ou protéase P4032, ou la subtilisine ou le Biofeed ou la trypsine.

7. Procédé selon l'une des revendications 4 à 6 dans lequel la protéase est la Prozyme.

8. Procédé selon l'une des revendications 1 à 3 **caractérisé en ce que** l'ester substantiellement énantiomériquement pur est de configuration *R* et l'enzyme utilisé est une lipase ou une estérase, à l'exception de l'enzeyme PLE (Pig Liver Esterase).

9. Procédé selon la revendication 8 dans lequel la lipase ou l'estérase est choisie parmi la lipase A Rhizopus, la lipase CE, la Chirazyme E1, Chirazyme L1, Chirazyme L2, Chirazyme L3, Chirazyme L4, Chirazyme L5, Chirazyme L6, Chirazyme L7, Chirazyme L8, la lipase GC, L3001, L3126, et la lipase pancréatique L115P.

10. Procédé selon l'une des revendications 8 ou 9 dans lequel la lipase ou l'estérase est choisie parmi la Chirazyme L2, L5, L7 et E1.

11. Procédé selon l'une quelconque des revendications 1 à 10 dans lequel l'énantiomère substantiellement pur est obtenu avec un excès énantiomérique supérieur à 80%.

12. Procédé selon l'une des revendications 1 à 11 dans lequel l'excès énantiomérique est supérieur à 90%.

13. Procédé selon l'une des revendications 1 à 12 dans lequel l'enzyme utilisé est non purifié, ou partiellement ou totalement purifié.

14. Procédé selon l'une des revendications 1 à 13, **caractérisé en ce que** le milieu réactionnel est en phase homogène.

15. Procédé selon l'une des revendications 1 à 13, **caractérisé en ce que** le milieu réactionnel est en phase hétérogène.

16. Procédé selon l'une des revendication 1 à 15 dans lequel l'enzyme est libre dans le milieu réactionnel.

17. Procédé selon l'une des revendications 1 à 15 dans lequel l'enzyme est immobilisé sur support.

18. Procédé selon l'une des revendications 1 à 17 **caractérisé en ce que** le milieu réactionnel a un pH compris entre 5 et 10.

19. Procédé selon l'une des revendications 1 à 18 **caractérisé en ce que** la température réactionnelle est comprise entre 4°C et 70°C.

20. Procédé selon l'une des revendications 1 à 19 **caractérisé en ce que** le temps de réaction est compris entre 1 heure et 120 heures.

21. Procédé selon l'une des revendications 1 à 20 **caractérisé en ce que** le temps de réaction est compris entre 10 heures et 60 heures.

22. Procédé selon l'une des revendications 1 à 21 **caractérisé en ce que** le milieu réactionnel comprend un ou plusieurs solvants aromatiques.

23. Procédé selon la revendication 22 dans lequel le solvant est le toluène ou le monochlorobenzène.

**24.** Utilisation d'un enzyme totalement ou partiellement purifié de type protéase pour l'obtention de l'énantiomère *S* de l'ester de formule (I) selon la revendication 1.

**25.** Utilisation de la protéase Prozyme pour l'obtention de l'énantiomère *S* de l'ester de formule (I) selon la revendication 1.

**26.** Procédé de préparation des composés de formule (F) selon le schéma réactionnel suivant :

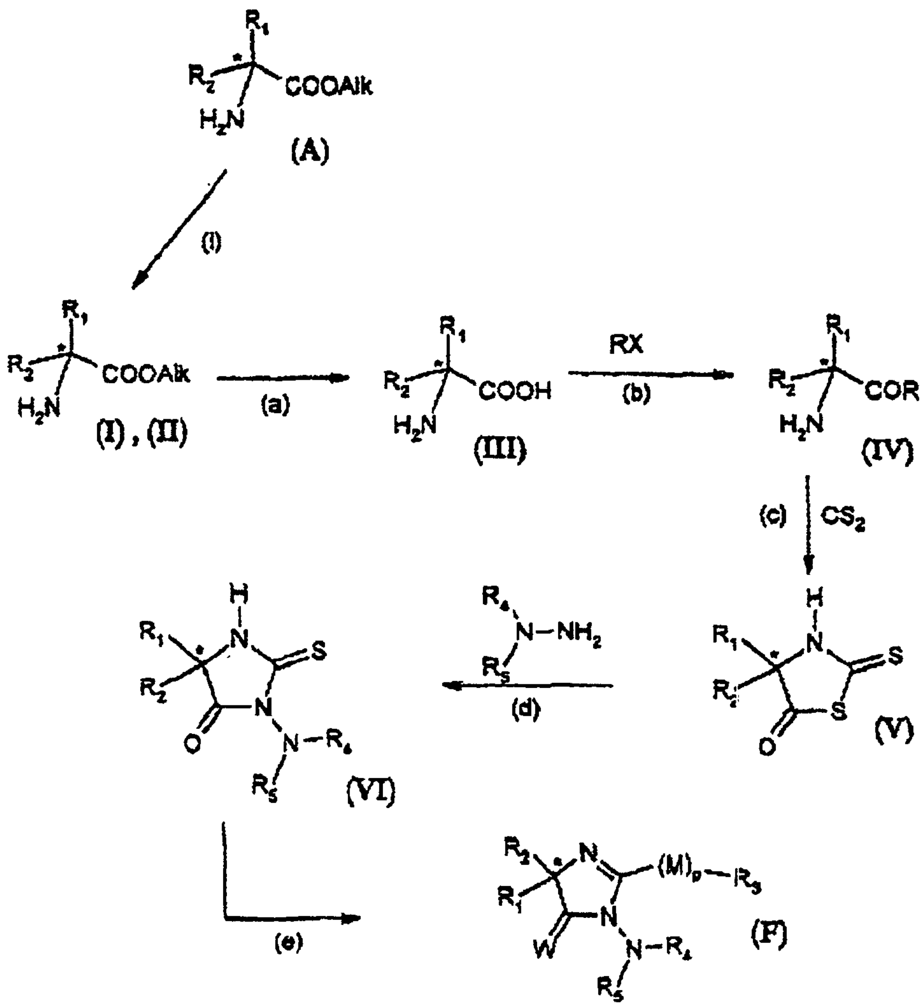


schéma dans lequel :

- $R_1$ représente le radical phényle et $R_2$ représente le radical méthyle ;
- W représente un atome d'oxygène ou de soufre, ou un groupe S=O ;
- M représente un atome d'oxygène ou de soufre, ou un radical $CH_2$, éventuellement halogéné ;
- p est un nombre entier égal à 0 ou 1 ;
- $R_3$ représente :
  - un hydrogène ou un radical $C_1$-$C_2$ alkyle éventuellement halogéné, lorsque p égale 0 ou $(M)_p$ est un radical $CH_2$,
  - un radical $C_1$-$C_2$ alkyle éventuellement halogéné, lorsque $(M)_p$ représente un atome d'oxygène ou de soufre ;
- représente :
  - l'atome d'hydrogène, ou

- un radical alkyle contenant de 1 à 6 atomes de carbone, ou
- un radical alkoxyalkyle, alkylthioalkyle, haloalkyle, cyanoalkyle, thiocyanatoalkyle, alcényle ou alcynyle contenant de 2 à 6 atomes de carbone, ou
- un radical dialkylaminoalkyle, alkoxycarbonylalkyle, ou N-alkylcarbamoylalkyle contenant de 3 à 6 atomes de carbone, ou
- un radical N,N-dialkylcarbamoylalkyle contenant de 4 à 8 atomes de carbone, ou
- un radical aryle, comprenant phényle, naphtyle, thiényle, furyle, pyridyle, pyrimidyle, pyridazinyle, pyrazinyle, benzothiényle, benzofuryle, quinolinyle, isoquinolinyle, ou méthylène dioxyphényle, éventuellement substitué par 1 à 3 groupements choisis parmi $R_6$, ou
- un radical arylalkyle, aryloxyalkyle, arylthioalkyle ou arylsulfonylalkyle, les termes aryle et alkyle ayant les définitions données ci-dessus ;

• $R_5$ représente :

- l'hydrogène, ou un radical alkyle, haloalkyle, alkylsulfonyle, haloalkylsulfonyle contenant de 1 à 6 atomes de carbone ou,
- un radical alkoxyalkyle, alkylthioakyle, acyle, alcényle, alcynyle, haloacyle, alkoxycarbonyle, haloalkoxycarbonyle, alkoxyalkylsulfonyle, cyanoalkylsulfonyle contenant de 2 à 6 atomes de carbone ou
- un radical alkoxyalkoxycarbonyle, alkylthioalkoxycarbonyle, cyanoalkoxycarbonyle contenant de 3 à 6 atomes de carbone ou,
- le radical formyle ou un radical cycloalkyle, alkoxyacyle, alkylthioacyle, cyanoacyle, alcénylcarbonyle, alcynylcarbonyle contenant de 3 à 6 atomes de carbone ou,
- un radical cycloalkylcarbonyle contenant de 4 à 8 atomes de carbone ou,
- un radical phényle; arylalkylcarbonyle, notamment phénylacétyle et phénylpropionyle, arylcarbonyle, notamment benzoyle, éventuellement substitué par 1 à 3 groupes parmi $R_6$, thiénylcarbonyle, furylcarbonyle, pyridylcarbonyle, benzyloxycarbonyle, furfuryloxycarbonyle, tetrahydrofurfuryloxycarbonyle, thiénylméthoxycarbonyle, pyridylméthoxycarbonyle, phénoxycarbonyle ou phénylthiolcarbonyle, le radical phényle étant lui-même éventuellement substitué par 1 à 3 groupement choisis parmi $R_6$, alkylthiolcarbonyle, haloalkylthiolcarbonyle, alkoxyalkylthiolcarbonyle, cyanoalkylthiolcarbonyle, benzylthiolcarbonyle, furfurylthiolcarbonyle, tétrahydrofurfurylthiolcarbonyle, thiénylméthylthiolcarbonyle, pyridylméthylthiolcarbonyle, ou arylsulfonyle ou
- un radical carbamoyle éventuellement mono ou disubstitué par :

  - un groupe alkyle ou haloalkyle contenant de 1 à 6 atomes de carbone,
  - un groupe cycloalkyle, alcényle ou alcynyle contenant de 3 à 6 atomes de carbone,
  - un groupe alkoxyalkyle, alkylthioalkyle ou cyanoalkyle contenant de 2 à 6 atomes de carbone ou,
  - un phényle éventuellement substitué par 1 à 3 groupement $R_6$;

- un groupement sulfamoyle éventuellement mono ou disubstitué par :

  - un groupe alkyle ou haloalkyle contenant de 1 à 6 atomes de carbone,
  - un groupe cycloalkyle, alcényle ou alcynyle contenant de 3 à 6 atomes de carbone,
  - un groupe alkoxyalkyle, alkylthioalkyle ou cyanoalkyle de 2 à 6 atomes de carbone ou
  - un phényle éventuellement substitué par 1 à 3 groupement $R_6$ ;

- un groupe alkylthioalkylsulfonyle contenant de 3 à 8 atomes de carbone ou cycloalkylsulfonyle contenant de 3 à 7 atomes de carbone ;
- $R_4$ et $R_5$ pris ensemble peuvent également former avec l'atome d'azote auquel ils sont attachés un groupe pyrrolidino, pipéridino, morpholino ou pipérazino éventuellement substitué par un radical alkyle contenant de 1 à 3 atomes de carbone.

• $R^6$ représente :

- un atome d'halogène ou
- un radical alkyle, haloalkyle, alkoxy, haloalkoxy, alkylthio, haloalkylthio ou alkylsulfonyle contenant de 1 à 6 atomes de carbone ou
- un radical cycloalkyle, halocycloalkyle, alcényloxy, alcynyloxy, alcénylthio, alcynylthio contenant de 3 à 6 atomes de carbone ou

- le groupe nitro ou cyano ou
- un radical amino éventuellement mono ou disubstitué par un radical alkyle ou acyle contenant de 1 à 6 atomes de carbone ou alkoxycarbonyle contenant de 2 à 6 atomes de carbone,
- un radical phényle, phénoxy ou pyridyloxy, ces radicaux étant éventuellement substitués par 1 à 3 groupements, identiques ou différents, choisis parmi $R_7$,

• $R_7$ représente :

- un atome d'halogène choisi parmi le fluor, le chlore, le brome, l'iode ou,
- un radical alkyle contenant de 1 à 6 atomes de carbone, ou
- un radical alkoxy ou alkylthio contenant de 1 à 6 atomes de carbone ou,
- un radical haloalkoxy ou haloalkylthio contenant de 1 à 6 atomes de carbone ou,
- un radical nitrile ou nitro ;

• R représente un radical hydroxy, alkoxy contenant de 1 à 6 atomes de carbone, benzyloxy, un radical amino, alkylamino ou dialkylamino, un radical alkylamino contenant de 1 à 6 atomes de carbone ;
• X représence un groupement partant tel qu'un atome d'halogène, choisi parmi chlore, brome et iode, ou un radical sulfate, ou alkylsulfonyloxy ou arylsulfonyloxy, et
• Alk est tel que défini dans la revendication 1,

schéma dans lequel :

- l'étape (i) est le procédé de préparation d'esters de l'α-phénylalanine substantiellement énantiomériquement purs de formules (I) et (II) selon l'une quelconque des revendications précédentes;
- l'étape (a) est une étape classique de saponification ;
- l'étape (b) est une réaction d'estérification classique ;
- l'étape (c) est une réaction de cyclisation réalisée par addition de sulfure de carbone, en milieu basique et connue de l'homme du métier ;
- l'étape (d) est réalisée par addition d'une amine de formule $N(R_4R_5)-NH_2$, en présence d'un catalyseur ;
- l'étape (e) est finalement réalisée par addition d'un composé de formule $R_3$-X, pour conduire au composé -S-$R_3$ correspondant, puis éventuellement oxydé, selon des techniques classiques pour conduire aux différents degrés d'oxydation de l'atome de soufre, ou encore traité par un composé de formule $R_3$-OH pour conduire au composé -O-$R_3$ correspondant.

## Patentansprüche

**1.** Verfahren zur Herstellung von α-Phenylalaninestern, die im wesentlichen enantiomer rein sind, der Formeln (I) und (II):

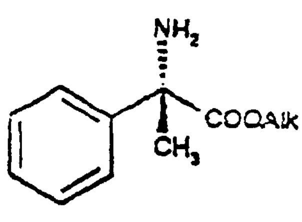

(I)

*S*-Isomer

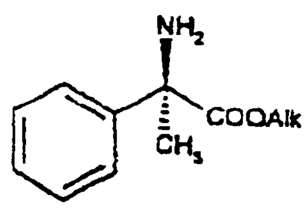

,

(II)

*R*-Isomer

in denen Alk geradkettiges oder verzweigtes Alkyl bedeutet, das 1 bis 6 Kohlenstoffatome aufweist, **dadurch gekennzeichnet, daß** ein Gemisch von Enantiomeren von α-Phenylalaninestern mit einem Enzym in Kontakt gebracht wird, bei dem es sich um eine Lipase, eine Protease oder eine Esterase mit Ausnahme des PLE-Enzyms (Pig Liver Esterase) handelt.

**2.** Verfahren nach Anspruch 1, wobei "Alk" Methyl bedeutet.

**3.** Verfahren nach Anspruch 1, wobei "Alk" Ethyl bedeutet.

**4.** Verfahren nach einem der Ansprüche 1 bis 3, **dadurch gekennzeichnet, daß** der im wesentlichen enantiomer reine Ester die S-Konfiguration aufweist und das verwendete Enzym eine Protease ist.

**5.** Verfahren nach Anspruch 4, wobei die Protease unter Biofeed, Prozym, Subtilisin, Subtilisin A, Trypsin, Protease PS, P5147 und P4032 ausgewählt wird.

**6.** Verfahren nach einem der Ansprüche 4 und 5, wobei es sich bei der Protease um eine Aspergillus-Protease, Prozyme oder Protease P4032, oder Subtilisin oder Biofeed oder Trypsin handelt.

**7.** Verfahren nach einem der Ansprüche 4 bis 6, wobei es sich bei der Protease um Prozyme handelt.

**8.** Verfahren nach einem der Ansprüche 1 bis 3, dadurch gekennzeichent, daß der im wesentlichen enantiomer reine Ester die R-Konfiguration aufweist und das verwendete Enzym eine Lipase oder eine Esterase mit Ausnahme des PLE-Enzyms (Pig Liver Esterase) ist.

**9.** Verfahren nach Anspruch 8, wobei die Lipase oder die Esterase unter Lipase A Rhizopus, Lipase CE, Chirazyme E1, Chirazyme L1, Chirazyme L2, Chirazyme L3, Chirazyme L4, Chirazyme L5, Chirazyme L6, Chirazyme L7, Chirazyme L8, Lipase GC, L3001, L3126 und der Pankreaslipase L115P ausgewählt wird.

**10.** Verfahren nach einem der Ansprüche 8 und 9, wobei die Lipase oder die Esterase unter Chirazyme L2, L5, L7 und E1 ausgewählt wird.

**11.** Verfahren nach einem der Ansprüche 1 bis 10, wobei das im wesentlichen reine Enantiomer mit einem enantiomeren Überschuß von mehr als 80 % erhalten wird.

**12.** Verfahren nach einem der Ansprüche 1 bis 11, wobei der enantiomere Überschuß größer als 90 % ist.

**13.** Verfahren nach einem der Ansprüche 1 bis 12, wobei das verwendete Enzym nicht gereinigt, teilweise gereinigt oder vollkommen gereinigt ist.

**14.** Verfahren nach einem der Ansprüche 1 bis 13, **dadurch gekennzeichnet, daß** das Reaktionsmedium in homogener Phase vorliegt.

**15.** Verfahren nach einem der Ansprüche 1 bis 13, **dadurch gekennzeichnet, daß** das Reaktionsmedium in heterogener Phase vorliegt.

**16.** Verfahren nach einem der Ansprüche 1 bis 15, wobei das Enzym in dem Reaktionsmedium in freier Form vorliegt.

**17.** Verfahren nach einem der Ansprüche 1 bis 15, wobei das Enzym auf einem Träger immobilisiert ist.

**18.** Verfahren nach einem der Ansprüche 1 bis 17, **dadurch gekennzeichnet, daß** das Reaktionsmedium einen pH-Wert im Bereich von 5 bis 10 aufweist.

**19.** Verfahren nach einem der Ansprüche 1 bis 18, **dadurch gekennzeichnet, daß** die Reaktionstemperatur im Bereich von 4 bis 70 °C liegt.

**20.** Verfahren nach einem der Ansprüche 1 bis 19, **dadurch gekennzeichnet, daß** die Reaktionsdauer im Bereich von 1 bis 120 h liegt.

**21.** Verfahren nach einem der Ansprüche 1 bis 20, **dadurch gekennzeichnet, daß** die Reaktionsdauer im Bereich von 1 bis 60 h liegt.

**22.** Verfahren nach einem der Ansprüche 1 bis 21, **dadurch gekennzeichnet, daß** das Reaktionsmedium ein oder mehrere aromatische Lösemittel enthält.

**23.** Verfahren nach Anspruch 22, wobei es sich bei dem Lösemittel um Toluol oder Monochlorbenzol handelt.

**24.** Verwendung eines vollkommen oder teilweise gereinigten Enzyms vom Proteasetyp zur Gewinnung des S-Enantiomers des Esters der Formel (I) nach Anspruch 1.

**25.** Verwendung der Protease Prozyme zur Gewinnung des S-Enantiomers des Esters der Formel (I) nach Anspruch 1.

**26.** Verfahren zur Herstellung der Verbindungen der Formel (F) nach dem folgenden Reaktionsschema

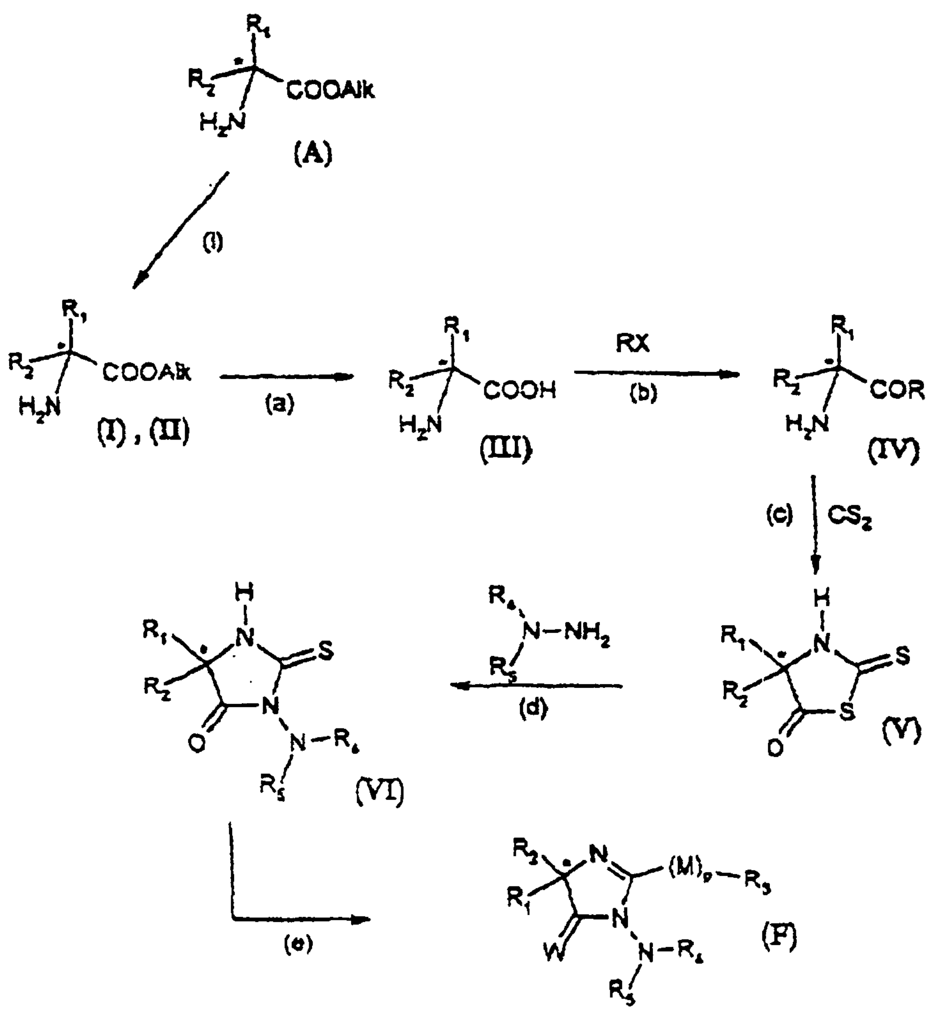


wobei in dem Reaktionsschema bedeuten:

- $R_1$ Phenyl und $R_2$ Methyl;
- W ein Sauerstoff- oder Schwefelatom oder eine Gruppe S=O;
- M ein Sauerstoff- oder Schwefelatom oder einen gegebenenfalls halogenierten Rest $CH_2$;
- p die ganze Zahl 0 oder 1,
- $R_3$
  - Wasserstoff oder gegebenenfalls halogeniertes $C_1$-$C_2$-Alkyl, wenn p gleich 0 ist oder $(M)_p$ einen Rest $CH_2$ bedeutet,
  - gegebenenfalls halogeniertes $C_1$-$C_2$-Alkyl, wenn $(M)_p$ ein Sauerstoff- oder Schwefelatom bedeutet,
- $R_4$
  - Wasserstoff oder

- Alkyl, das 1 bis 6 Kohlenstoffatome enthält, oder
- Alkoxyalkyl, Alkylthioalkyl, Halogenalkyl, Cyanoalkyl, Thiocyanatoalkyl, Alkenyl oder Alkinyl, das 2 bis 6 Kohlenstoffatome enthält, oder
- Dialkylaminoalkyl, Alkoxycarbonylalkyl oder N-Alkylcarbamoylalkyl, das 3 bis 6 Kohlenstoffatome enthält, oder
- N,N-Dialkylcarbamoylalkyl, das 4 bis 8 Kohlenstoffatome enthält, oder
- einen Arylrest einschließlich Phenyl, Naphthyl, Thienyl, Furyl, Pyridyl, Pyrimidyl, Pyridazinyl, Pyrazinyl, Benzothienyl, Benzofuryl, Chinolinyl, Isochinolinyl und Methylendioxyphenyl, der gegebenenfalls mit 1 bis 3 Gruppen substituiert ist, die unter R6 ausgewählt werden, oder
- Arylalkyl, Aryloxyalkyl, Arylthioalkyl oder Arylsulfonylalkyl, wobei die Ausdrücke Aryl und Alkyl den weiter unten angegebenen Definitionen entsprechen;

• $R_5$

- Wasserstoff oder Alkyl, Halogenalkyl, Alkylsulfonyl, Halogenalkylsulfonyl, das 1 bis 6 Kohlenstoffatome enthält oder
- Alkoxyalkyl, Alkylthioalkyl, Acyl, Alkenyl, Alkinyl, Halogenacyl, Alkoxycarbonyl, Halogenalkoxycalbonyl, Alkoxyalkylsulfonyl, Cyanoalkylsulfonyl, das 2 bis 6 Kohlenstoffatome enthält, oder
- Alkoxyalkoxycarbonyl, Alkylthioalkoxycarbonyl, Cyanoalkoxycarbonyl, das 3 bis 6 Kohlenstoffatome enthält, oder
- Formyl oder Cycloalkyl, Alkoxyacyl, Alkylthioacyl, Cyanoacyl, Alkenylcarbonyl, Alkinylcarbonyl, das 3 bis 6 Kohlenstoffatome enthält, oder
- Cycloalkylcarbonyl, das 4 bis 8 Kohlenstoffatome enthält, oder
- Phenyl; Arylalkylcarbonyl, insbesondere Phenylacetyl und Phenylpropionyl, Arylcarbonyl, insbesondere Benzoyl, das gegebenenfalls mit 1 bis 3 Gruppen substituiert ist, die unter $R_6$ ausgewählt werden, Thienylcarbonyl, Furylcarbonyl, Pyridylcarbonyl, Benzyloxycarbonyl, Furfuryloxycarbonyl, Tetrahydrofurfuryloxycarbonyl, Thienylmethoxycarbonyl, Pyridylmethoxycarbonyl, Phenoxycarbonyl oder Phenylthiolcarbonyl, wobei der Phenylrest selbst gegebenenfalls mit 1 bis 3 Gruppen substituiert ist, die unter $R_6$ ausgewählt werden, Alkylthiolcarbonyl, Halogenalkylthiolcarbonyl, Alkoxyalkylthiolcarbonyl, Cyanoalkylthiolcarbonyl, Benzylthiolcarbonyl, Furfurylthiolcarbonyl, Tetrahydrofurfurylthiolcarbonyl, Thienylmethylthiolcarbonyl, Pyridylmethylthiolcarbonyl oder Arylsulfonyl oder
- Carbamoyl, das gegebenenfalls mono- oder disubstituiert ist mit

  * einer Alkyl- oder Halogenalkylgruppe, die 1 bis 6 Kohlenstoffatome enthält,
  * einer Cycloalkyl-, Alkenyl- oder Alkinylgruppe, die 3 bis 6 Kohlenstoffatome enthält,
  * einer Alkoxyalkyl-, Alkylthioalkyl- oder Cyanoalkylgruppe, die 2 bis 6 Kohlenstoffatome enthält,
  * einem Phenylrest, der gegebenenfalls mit 1 bis 3 Gruppen $R_6$ substituiert ist,

- Sulfamoyl, das gegebenenfalls mono- oder disubstituiert ist mit

  * einer Alkyl- oder Halogenalkylgruppe, die 1 bis 6 Kohlenstoffatome enthält,
  * einer Cycloalkyl-, Alkenyl- oder Alkinylgruppe, die 3 bis 6 Kohlenstoffatome enthält,
  * einer Alkoxyalkyl-, Alkylthioalkyl- oder Cyanoalkylgruppe, die 2 bis 6 Kohlenstoffatome enthält,
  * einem Phenylrest, der gegebenenfalls mit 1 bis 3 Gruppen $R_6$ substituiert ist,

- Alkylthioalkylsulfonyl, das 3 bis 8 Kohlenstoffatome enthält, oder Cycloalkylsulfonyl, das 3 bis 7 Kohlenstoffatome enthält
- $R_4$ und $R_5$ Reste, die außerdem zusammen mit dem Stickstoffatom, mit dem sie verbunden sind, eine Pyrrolidino-, Piperidino-, Morpholino- oder Piperazinogruppe bilden können, die gegebenenfalls mit einem Alkylrest substituert ist, der 1 bis 3 Kohlenstoffatome enthält;

• $R_6$

- ein Halogenatom oder
- Alkyl, Halogenalkyl, Alkoxy, Halogenalkoxy, Alkylthio, Halogenalkylthio oder Alkylsulfonyl, das 1 bis 6 Kohlenstoffatome enthält, oder
- Cycloalkyl, Halogencycloalkyl, Alkenyloxy, Alkinyloxy, Alkenylthio, Alkinylthio, das 3 bis 6 Kohlenstoffatome enthält, oder

- eine Nitro- oder Cyanogruppe oder
- eine Aminogruppe, die gegebenenfalls mit einem Alkyl- oder Acylrest, der 1 bis 6 Kohlenstoffatome enthält, oder einem Alkoxycarbonylrest, der 2 bis 6 Kohlenstoffatome enthält, mono- oder disubstituiert ist,
- Phenyl, Phenoxy oder Pyridyloxy, wobei diese Reste gegebenenfalls mit 1 bis 3 gleichen oder verschiedenen Gruppensubstituiert sind, die unter $R_7$ ausgewählt werden;

• $R_7$

- ein Halogenatom, das unter Fluor, Chlor, Brom, Iod ausgewählt wird, oder
- Alkyl, das 1 bis 6 Kohlenstoffatome enthält,
- Alkoxy oder Alkylthio, das 1 bis 6 Kohlenstoffatome enthält, oder
- Halogenalkoxy oder Halogenalkylthio, das 1 bis 6 Kohlenstoffatome enthält, oder
- eine Nitril- oder Nitrogruppe;

• R Hydroxy, Alkoxy, das 1 bis 6 Kohlenstoffatome enthält, Benzyloxy, Amino, Alkylamino oder Dialkylamino, wobei ein Alkylaminorest 1 bis 6 Kohlenstoffatome enthält;
• X eine Abgangsgruppe, wie ein Halogenatom, das unter Chlor, Brom und Iod ausgewählt wird, oder Sulfat oder Alkylsulfonyloxy oder Arylsulfonyloxy und
• Alk einen Rest, der wie in Anspruch 1 definiert ist,

wobei in dem Schema

- der Schritt (i) das Verfahren zur Herstellung der im wesentlichen enantiomer reinen α-Phenylalaninester der Formeln (I) und (II) nach einem der vorhergehenden Ansprüchen darstellt;
- Schritt (a) eine herkömmliche Verseifung ist;
- Schritt (b) eine herkömmliche Veresterung ist;
- Schritt (c) eine dem Fachmann bekannte Cyclisierungsreaktion ist, die unter Zugabe von Schwefelkohlenstoff in basischem Medium durchgeführt wird;
- Schritt (d) unter Zugabe eines Amins der Formel $N(R_4R_5)-NH_2$ in Gegenwart eines Katalysators durchgeführt wird;
- Schritt (e) schließlich durchgeführt wird, indem eine Verbindung der Formel $R_3$-X zugegeben wird, um die entsprechende Verbindung -S-$R_3$ zu erhalten, die dann gegebenenfalls nach herkömmlichen Techniken oxidiert wird, um ein Schwefelatom in den verschiedenen Oxidationsstufen zu erhalten, oder die auch mit einer Verbindung der Formel $R_3$-OH behandelt wird, um die entsprechende Verbindung -O-$R_3$ zu erhalten.

## Claims

1. Method for preparing substantially enantiomerically pure α-phenylalanine esters of formulae (I) and (II):

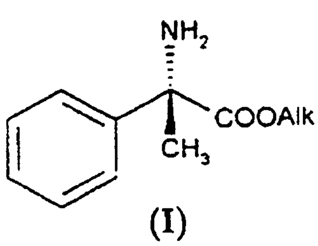
(I)
*S* isomer

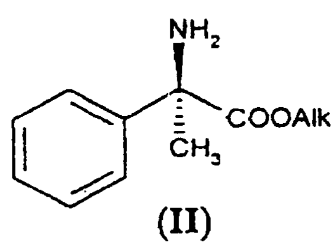
(II)
*R* isomer

in which Alk represents a linear or branched alkyl radical containing from 1 to 6 carbon atoms, **characterized in that** a mixture of enantiomers of α-phenylalanine esters is brought into contact with an enzyme which is a lipase, a protease or an esterase, with the exception of the enzyme PLE (Pig Liver Esterase).

2. Method according to Claim 1, in which "Alk" represents the methyl radical.

3. Method according to Claim 1, in which "Alk" represents the ethyl radical.

4. Method according to one of Claims 1 to 3, **characterized in that** the substantially enantiomerically pure ester is of *S* configuration and the enzyme used is a protease.

5. Method according to Claim 4, in which the protease is chosen from Biofeed, Prozyme, Subtilisin, Subtilisin A, Trypsin, protease PS, P5147 and P4032.

6. Method according to either of Claims 4 and 5, in which the protease is an aspergillus protease, Prozyme or protease P4032, or subtilisin or Biofeed or trypsin.

7. Method according to one of Claims 4 to 6, in which the protease is Prozyme.

8. Method according to one of Claims 1 to 3, **characterized in that** the substantially enantiomerically pure ester is of *R* configuration and the enzyme used is a lipase or an esterase, with the exception of the enzyme PLE (Pig Liver Esterase).

9. Method according to Claim 8, in which the lipase or the esterase is chosen from lipase A Rhizopus, lipase CE, Chirazyme E1, Chirazyme L1, Chirazyme L2, Chirazyme L3, Chirazyme L4, Chirazyme L5, Chirazyme L6, Chirazyme L7, Chirazyme L8, lipase GC, L3001, L3126 and pancreatic lipase L115P.

10. Method according to either of Claims 8 and 9, in which the lipase or esterase is chosen from Chirazyme L2, L5, L7 and E1.

11. Method according to any one of Claims 1 to 10, in which the substantially pure enantiomer is obtained with an enantiomeric excess greater than 80%.

12. Method according to one of Claims 1 to 11, in which the enantiomeric excess is greater than 90%.

13. Method according to one of Claims 1 to 12, in which the enzyme used is unpurified, or partially or completely purified.

14. Method according to one of Claims 1 to 13, **characterized in that** the reaction medium is in homogeneous phase.

15. Method according to one of Claims 1 to 13, **characterized in that** the reaction medium is in heterogeneous phase.

16. Method according to one of Claims 1 to 15, in which the enzyme is free in the reaction medium.

17. Method according to one of Claims 1 to 15, in which the enzyme is immobilized on a support.

18. Method according to one of Claims 1 to 17, **characterized in that** the reaction medium has a pH of between 5 and 10.

19. Method according to one of Claims 1 to 18, **characterized in that** the reaction temperature is between 4°C and 70°C.

20. Method according to one of Claims 1 to 19, **characterized in that** the reaction time is between 1 hour and 120 hours.

21. Method according to one of Claims 1 to 20, **characterized in that** the reaction time is between 10 hours and 60 hours.

22. Method according to one of Claims 1 to 21, **characterized in that** the reaction medium comprises one or more aromatic solvents.

23. Method according to Claim 22, in which the solvent is toluene or monochlorobenzene.

24. Use of a completely or partially purified enzyme of the protease type for producing the *S* enantiomer of the ester of formula (I) according to Claim 1.

25. Use of the protease Prozyme for producing the *S* enantiomer of the ester of formula (I) according to Claim 1.

26. Method of preparing the compounds of formula (F) according to the following reaction scheme:

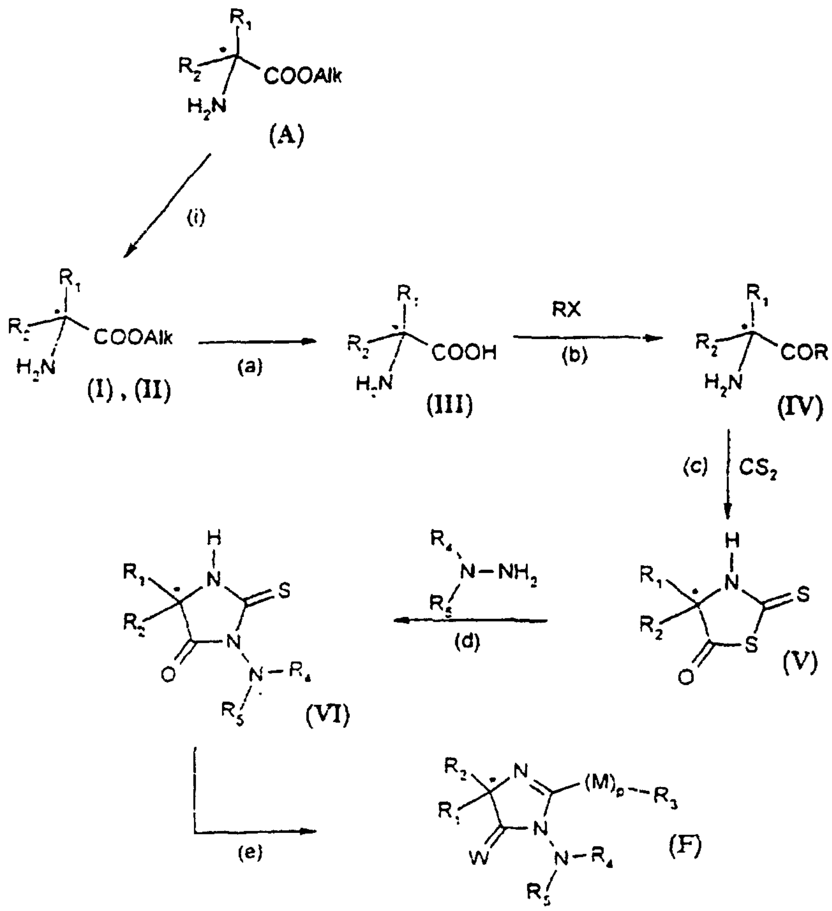


in which scheme:

- $R_1$ represents the phenyl radical and $R_2$ represents the methyl radical;
- W represents an oxygen or sulphur atom, or a group S=O;
- M represents an oxygen or sulphur atom, or a $CH_2$ radical, optionally halogenated;
- p is an integer equal to 0 or 1;
- $R_3$ represents:
  - a hydrogen or an optionally halogenated $C_1$-$C_2$ alkyl radical, when p equals 0 or $(M)_p$ is a $CH_2$ radical,
  - an optionally halogenated $C_1$-$C_2$ alkyl radical, when $(M)_p$ represents an oxygen or sulphur atom;
- $R_4$ represents:
  - the hydrogen atom, or
  - an alkyl radical containing from 1 to 6 carbon atoms, or
  - an alkoxyalkyl, alkylthioalkyl, haloalkyl, cyanoalkyl, thiocyanatoalkyl, alkenyl or alkynyl radical containing from 2 to 6 carbon atoms, or
  - a dialkylaminoalkyl, alkoxycarbonylalkyl or N-alkylcarbamoylalkyl radical containing from 3 to 6 carbon atoms, or
  - an N,N-dialkylcarbamoylalkyl radical containing from 4 to 8 carbon atoms, or
  - an aryl radical comprising phenyl, naphthyl, thienyl, furyl, pyridyl, pyrimidyl, pyridazinyl, pyrazinyl, benzothienyl, benzofuryl, quinolyl, isoquinolyl, or methylenedioxyphenyl, optionally substituted with 1 to 3 groups

chosen from $R_6$, or
- an arylalkyl, aryloxyalkyl, arylthioalkyl or arylsulphonylalkyl radical, the terms aryl and alkyl having the definitions given above;

• $R_5$ represents:

- hydrogen, or an alkyl, haloalkyl, alkylsulphonyl or haloalkylsulphonyl radical containing from 1 to 6 carbon atoms, or
- an alkoxyalkyl, alkylthioalkyl; acyl, alkenyl, alkynyl, haloacyl, alkoxycarbonyl, haloalkoxycarbonyl, alkoxyalkylsulphonyl or cyanoalkylsulphonyl radical containing from 2 to 6 carbon atoms, or
- an alkoxyalkoxycarbonyl, alkylthioalkoxycarbonyl or cyanoalkoxycarbonyl radical containing from 3 to 6 carbon atoms, or
- the formyl radical or a cycloalkyl, alkoxyacyl, alkylthioacyl, cyanoacyl, alkenylcarbonyl or alkynylcarbonyl radical containing from 3 to 6 carbon atoms, or
- a cycloalkylcarbonyl radical containing from 4 to 8 carbon atoms, or
- a radical phenyl; arylalkylcarbonyl, in particular phenylacetyl and phenylpropionyl, arylcarbonyl, in particular benzoyl, optionally substituted with 1 to 3 groups from $R_6$, thienylcarbonyl, furylcarbonyl, pyridylcarbonyl, benzyloxycarbonyl, furfuryloxycarbonyl, tetrahydrofurfuryloxycarbonyl, thienylmethoxycarbonyl, pyridylmethoxycarbonyl, phenoxycarbonyl or phenylthiocarbonyl, the phenyl radical being itself optionally substituted with 1 to 3 groups chosen from $R_6$, alkylthiocarbonyl, haloalkylthiocarbonyl, alkoxyalkylthiocarbonyl, cyanoalkylthiocarbonyl, benzylthiocarbonyl, furfurylthiocarbonyl, tetrahydrofurfurylthiocarbonyl, thienylmethylthiocarbonyl, pyridylmethylthiocarbonyl or arylsulphonyl, or
- a carbamoyl radical optionally mono- or disubstituted with:
- an alkyl or haloalkyl group containing from 1 to 6 carbon atoms,
- a cycloalkyl, alkenyl or alkynyl group containing from 3 to 6 carbon atoms,
- an alkoxyalkyl, alkylthioalkyl or cyanoalkyl group containing from 2 to 6 carbon atoms, or
- a phenyl optionally substituted with 1 to 3 groups $R_6$;
- a sulphamoyl group optionally mono- or disubstituted with:
- an alkyl or haloalkyl group containing from 1 to 6 carbon atoms,
- a cycloalkyl, alkenyl or alkynyl group containing from 3 to 6 carbon atoms,
- an alkoxyalkyl, alkylthioalkyl or cyanoalkyl group containing from 2 to 6 carbon atoms, or
- a phenyl optionally substituted with 1 to 3 groups $R_6$;
- an alkylthioalkylsulphonyl group containing from 3 to 8 carbon atoms or a cycloalkylsulphonyl group containing from 3 to 7 carbon atoms;
- $R_4$ and $R_5$, taken together, can also form with the nitrogen atom to which they are attached a pyrrolidino, piperidino, morpholinc or piperazino group optionally substituted with an alkyl radical containing from 1 to 3 carbon atoms,

• $R_6$ represents:

- a halogen atom, or
- an alkyl, haloalkyl, alkoxy, haloalkoxy, alkylthio, haloalkylthio or alkylsulphonyl radical containing from 1 to 6 carbon atoms, or
- a cycloalkyl, halocycloalkyl, alkenyloxy, alkynyloxy, alkenylthio or alkynylthio radical containing from 3 to 6 carbon atoms, or
- the nitro or cyano group, or
- an amino radical optionally mono- or disubstituted with an alkyl or acyl radical containing from 1 to 6 carbon atoms or an alkoxycarbonyl radical containing from 2 to 6 carbon atoms,
- a phenyl, phenoxy or pyridyloxy radical, it being possible for these radicals to be optionally substituted with 1 to 3 groups, which are identical or different, chosen from $R_7$,

• $R_7$ represents:

- a halogen atom chosen from fluorine, chlorine, bromine or iodine, or
- an alkyl radical containing from 1 to 6 carbon atoms, or
- an alkoxy or alkylthio radical containing from 1 to 6 carbon atoms, or
- a haloalkoxy or haloalkylthio radical containing from 1 to 6 carbon atoms, or
- a nitrile or nitro radical;

- R represents a hydroxyl radical, an alkoxy radical containing from 1 to 6 carbon atoms, a benzyloxy radical, an amino, alkylamino or dialkylamino radical or an alkylamino radical containing from 1 to 6 carbon atoms;
- X represents a leaving group such as a halogen atom, chosen from chlorine, bromine and iodine, or a sulphate, or alkylsulphonyloxy or arylsulphonyloxy radical, and
- Alk is as defined in Claim 1,

in which scheme:

- step (i) is the method for preparing substantially enantiomerically pure α-phenylalanine esters of formulae (I) and (II) according to any one of the preceding claims;
- step (a) is a conventional saponification step;
- step (b) is a conventional esterification reaction;
- step (c) is a cyclization reaction performed by addition of carbon sulphide, in basic medium and known to a person skilled in the art;
- step (d) is carried out by addition of an amine of formula $N(R_4R_5)-NH_2$, in the presence of a catalyst;
- step (e) is finally carried out by addition of a compound of formula $R_3$-X so as to give the corresponding compound -S-$R_3$, and then optionally oxidized according to conventional techniques to give the different oxidation states of the sulphur atom, or alternatively treated with a compound of formula $R_3$-OH in order to give the corresponding compound -O-$R_3$.